(19) Europäisches Patentamt
European Patent Office
Office européen des brevets

(11) Veröffentlichungsnummer: **0 252 361 A2**

(12) **EUROPÄISCHE PATENTANMELDUNG**

(21) Anmeldenummer: **87108952.0**

(51) Int. Cl.⁴: **C07H 3/02**

(22) Anmeldetag: **23.06.87**

(30) Priorität: **05.07.86 DE 3622643**

(43) Veröffentlichungstag der Anmeldung: **13.01.88 Patentblatt 88/02**

(84) Benannte Vertragsstaaten: **CH DE FR GB LI NL**

(71) Anmelder: **BASF Aktiengesellschaft**
**Carl-Bosch-Strasse 38**
**D-6700 Ludwigshafen(DE)**

(72) Erfinder: **Dobler, Walter, Dr.**
**Wilhelm-Blum-Strasse 4**
**D-6900 Heidelberg(DE)**
Erfinder: **Ernst, Hansgeorg, Dr.**
**Bruesseler Ring 38**
**D-6700 Ludwigshafen(DE)**
Erfinder: **Paust, Joachim, Dr.**
**Ringstrasse 3**
**D-6708 Neuhofen(DE)**

# (54) Verbessertes Verfahren zur Epimerisierung von Zuckern, insbesondere von D-Arabinose zu D-Ribose.

(57) Verbessertes Verfahren zur Epimerisierung von Pentosen und Hexosen durch Erhitzen von in einem Lösungsmittel gelöstem Zucker in Gegenwart einer Molybdän(VI)-Verbindung, das dadurch gekennzeichnet ist, daß man zur Herstellung eines Zuckers der Formeln Ia oder Ib mit cis-ständigen OH-Gruppen in 2-oder 3-Stellung des Zuckers

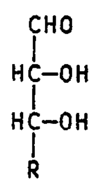
(Ia)

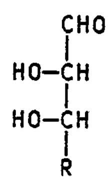
(Ib)

in der R für einen der Reste

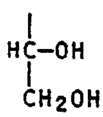
,
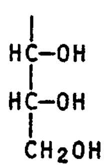
oder
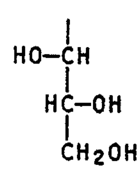

steht,
eine homogene Lösung des entsprechenden Zuckers der Formeln IIa oder IIb

EP 0 252 361 A2

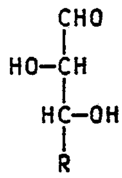

(IIa)

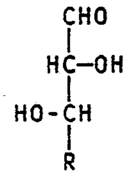

(IIb)

in Gegenwart von 30 bis 200 Mol.%, bezogen auf eingesetzten Zucker, an einem gegebenenfalls Kristallwasser enthaltenden Metallsalz der Formel (III)

$MeX_2$ (III),

in der Me für Mg, Ca, Sr, Ba und Zn steht und X für Cl oder Br steht, und in Gegenwart von 2 bis 20 Mol.%, bezogen auf den eingesetzten Zucker einer Molybdän(VI)-Verbindung auf Temperaturen von 75 bis 100°C erhitzt.

Das Verfahren hat besondere Bedeutung für die Herstellung der als Zwischenprodukt für Vitamin $B_2$ benötigten D-Ribose.

# Verbessertes Verfahren zur Epimerisierung von Zuckern, insbesondere von D-Arabinose zu D-Ribose

Die Erfindung betrifft ein verbessertes Verfahren zur Epimerisierung von Zuckern mit trans-ständigen OH-Gruppen in 2-und 3-Stellung des Zuckers. Besondere Bedeutung hat dieses Verfahren für die Herstellung der für die Synthese von Vitamin $B_2$ benötigten D-Ribose durch Epimerisieren von D-Arabinose.

Es ist bekannt, daß D-Ribose durch Extraktion aus Naturstoffen, durch Fermentation eines Mikroorganismus oder durch chemische Synthese aus Furan oder Glucose hergestellt werden kann. Alle diese Verfahren zeichnen sich jedoch dadurch aus, daß sie kompliziert sind und eine geringe Ausbeute ergeben.

Die Herstellung von D-Ribose wurde technisch im allgemeinen nach einem Verfahren durchgeführt, bei dem D-Glucose zur Bildung von D-Arabonsäure mit Sauerstoff in einer wäßrigen Alkali-Lösung oxidiert und diese in Form eines Metallsalzes wie z.B. von Quecksilber oder Zinksalz abgetrennt, zu D-Ribonolacton lactonisiert und mit Natriumamalgam zu D-Ribose reduziert wurde. Die Erwärmung von D-Arabonsäure in einer wäßrigen Alkalilösung ergibt eine Mischung, die ein Gleichgewichtsverhältnis von D-Arabonsäure zu D-Ribonsäure von 70:30 aufweist. Es ist hierbei unmöglich, einen Anteil von D-Ribonsäure zu erhalten, der 30 % übersteigt. Schwierigkeiten ergaben sich für das Verfahren außerdem durch die Verwendung der großen Mengen an Quecksilber für die Amalgam-Reduktion.

Bilik et al. berichteten über die Epimerisierbarkeit von verschiedenen Sacchariden in wäßriger Lösung in Gegenwart eines Molybdänsäure-Katalysators, einschließlich der Epimerisierbarkeit von L-Arabinose zu L-Ribose (vgl. tschechisches Patent Nr. 149 472; Chemical Abstracts 81, 78 189 K).

Basierend auf dieser Erkenntnis wurde ein Verfahren entwickelt, bei dem D-Gluconsäure anstatt zu D-Arabonsäure zu D-Arabinose oxidiert wurde. Als Oxidationsmittel diente Hypochlorit. D-Arabinose wird dann in wäßriger Lösung in Gegenwart eines Molybdänkatalysators zu D-Ribose epimerisiert (vgl. japanische offengelegte Patentanmeldung Nr. 164 699, 1980 bzw. EP 20 959 und US 4 355 158). Dieses Verfahren erreicht ein Epimerisierungsverhältnis (Anteil von Ribose in einer Gleichgewichtsmischung) von nur ungefähr 25 %. Dennoch ist dieses Verfahren den zuvor beschriebenen überlegen, da hierbei kein Quecksilber verwendet wird und weniger Verfahrensschritte notwendig sind. In einer Verfahrensvariante wird ein Großteil der Arabinose kristallin abgetrennt und wieder in die Epimerisierung zurückgeführt. Um die Abtrennung der Molybdänsäure aus der Epimerisierungslösung zu erleich tern, wurde die Verwendung eines Molybdänsäure-tragenden Ionenaustauscher-Harzes anstelle der Molybdänsäure (vgl. Japanische Patentveröffentlichung Nr. 40 700/1981), oder die Verwendung einer Molybdänsäure tragenden Ionenaustauscher-Faser, (vgl. japanische offengelegte Patentanmeldung Nr. 76 894/1980), beschrieben. Das Epimerisierungsverhältnis von D-Arabinose/D-Ribose beträgt 69,4/30,6. Die japanische offengelegte Patentanmeldung Nr. 54 197/1982 offenbart ein Epimerisierungsverhältnis von 27,2 % D-Ribose.

Es ist weiterhin bekannt, daß durch Erhitzen von L-Arabinose in Dimethylformamid in Gegenwart von Dioxo-bis(2,4-pentadionato-0,0')-molybdän (VI) eine Epimerisierung von 36 % der L-Arabinose zu L-Ribose erfolgt (vgl. Abe et al. in Chemical und Pharmaceutical Bulletin, 28 (1980), Seite 1324).

Eine weitere Verbesserung der Riboseselektivität wurde dadurch erreicht, daß man Borverbindungen in 2-3fach molarer Menge dem Epimiersierungsgemisch zusetzte (vgl. JP 1890.976/83, JP 223.187/83 und DE-OS 34 37 571). In wäßriger Lösung wurde hierbei ein Epimerisierungsgleichgewicht von ca. 60 %, in nichtwäßriger Lösung von bis zu 94 % erreicht. Nachteilig an diesem Verfahren ist, daß die Borsäure nicht auf eine für die Vitamin-$B_2$-Herstellung tolerierbare Menge abgetrennt werden kann, ohne daß Ribose und Arabinose mit entfernt werden, d.h., daß die Ausbeute an Gesamtzuckern stark abfällt mit jeder Maßnahme zur Abtrennung der Borsäure. Außerdem kann die unumgesetzte Arabinose aus borsäurehaltiger Lösung nicht von Ribose abgetrennt und wieder eingesetzt werden.

Es war daher die Aufgabe der Erfindung, das Verfahren zur Herstellung einer D-Ribose-haltigen Lösung aus einer D-Arabinose-haltigen Lösung durch Epimerisierung in Gegenwart einer Molybdän(VI)-Verbindung so zu verbessern, daß das Epimerisierungsgleichgewicht stark zugunsten der Ribose verschoben wird, ohne daß die Zuckerausbeute durch die Abtrennung des Hilfsstoffes stark vermindert wird. Es war weiterhin die Aufgabe der Erfindung, ein von den Nachteilen des Standes der Technik freies Epimerisierungsverfahren zu entwickeln, das auch für die Epimerisierung anderer Pentosen und Hexosen geeignet ist.

Es wurde nun überraschenderweise gefunden, daß das Epimerisierungsgleichgewicht für die Epimerisierung von Arabinose zu Ribose in Gegenwart einer Molybdän(VI)-Verbindung weitgehend zugunsten der Ribose verschoben werden kann, wenn man die Epimerisierung der Arabinoselösung zusätzlich in Gegenwart von 30 bis 200 Mol.% des Bromids oder Chlorids eines Erdalkalimetalles oder von Zink, bezogen auf eingesetzte Arabinose, durchführt. Überraschend hieran war auch, daß die als Katalysator benötigte

Molyb dän(VI)-Verbindung während der Epimerisierung trotz des großen Überschusses an Erdalkali-oder Zink-ionen nicht als schwerlösliches Molybdat ausfällt, jedoch nach der Epimerisierung die Metallsalze und die Molybdän-Verbindung durch Zugabe von Natrium-oder Ammoniumcarbonat nahezu quantitativ als -schwerlöslicher Niederschlag ausgefällt werden können, ohne daß Zucker mit ausgefällt werden.

Dieser Metallsalzzusatz bringt auch bei der Epimerisierung anderer Pentosen und Hexosen eine Verschiebung des Epimerisierungsgleichgewichts mit sich und zwar jeweils zugunsten des Zuckers mit der größeren Anzahl der cis-ständigen OH-Gruppen. Dies läßt vermuten, daß die Verschiebung des Epimerisierungsgleichgewichtes im wesentlichen auf die Bildung von Zucker-Metallsalz-Komplexen zurückzuführen ist. Glücklicherweise sind die Komplexe nicht sehr stabil, so daß die Metallsalze anschließend wieder gut abtrennbar sind.

Gegenstand der Erfindung ist daher ein verbessertes Verfahren zur Epimerisierung von Pentosen und Hexosen durch Erhitzen von in einem Lösungsmittel gelöstem Zucker in Gegenwart einer Molybdän(VI)-Verbindung, das dadurch gekennzeichnet ist, daß man zur Herstellung eines Zuckers der Formeln Ia oder Ib mit cis-ständigen OH-Gruppen in 2-und 3-Stellung des Zuckers

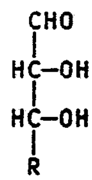

(Ia)

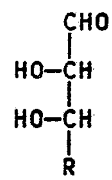

(Ib)

in der R für einen der Reste

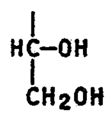

,

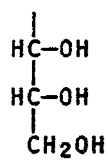

oder

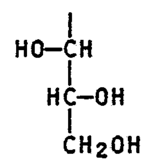

steht,
eine homogene Lösung des entsprechenden Zuckers der Formeln IIa oder IIb

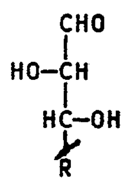

(IIa)

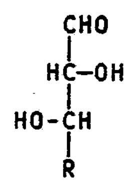

(IIb)

in Gegenwart von 30 bis 200 Mol.%, vorzugsweise 80 bis 120 Mol.%, bezogen auf den eingesetzten Zucker, an einem gegebenenfalls Kristallwasser enthaltenden Metallsalz der Formel (III)

$MeX_2$ (III),

in der Me für Mg, Ca, Sr, Ba und Zn und X für Cl oder Br steht, und in Gegenwart von 2 bis 20, vorzugsweise 8 bis 12 Mol.%, bezogen auf den eingesetzten Zucker, einer Molybdän(VI)-Verbindung auf Temperaturen von 75 bis 100°C erhitzt.

Besonders vorteilhaft gestaltet sich das Verfahren, wenn man aus der bei der Epimerisierung erhaltenen Reaktionslösung den Molybdän-Katalysator und das Metallsalz durch Zugabe eines Alkali-oder Ammoniumcarbonats gemeinsam als schwer löslichen Niederschlag ausfällt, die Neutralsalze (vorwiegend Alkali-bzw. Ammoniumchloride) in bekannter Weise durch Elektrodialyse entfernt und anschließend den unumgesetzten Zucker in an sich bekannter Weise auskristallisiert, oder wenn man das erhaltene rohe Reaktionsgemisch in Form einer wäßrigen Lösung direkt an einem stark sauren Kationenaustauscher, dessen Harz mit den bei der Epimerisierung verwendeten Metallionen beladen ist, chromatographisch fraktioniert. Besonders vorteilhaft bei der chromatographischen Trennung ist, daß die Molybdänverbindung aus den sie enthaltenden Fraktionen durch Ausfällen mit NaOH oder $NH_4OH$ praktisch quantitativ als Molybdat ausgefällt und dadurch zurückgewonnen und wiederverwendet werden kann. Durch Fällen mit $Na_2CO_3$-oder $(NH_4)_2CO_3$-Lösung können die Molybdänverbindung und der $MeCl_2$-Zusatz gemeinsam ausgefällt werden. Beide Maßnahmen ermöglichen, daß die Schwermetallbelastung der Abwässer nur ganz minimal ist. Der mit den Carbonaten ausgefällte Niederschlag kann mit 50 bis 200 Mol.% der entsprechenden Säure HX (X = Cl oder Br) in situ leicht wieder aufgearbeitet und die Metalle erneut bei der Epimerisierung eingesetzt werden.

Ganz besondere Bedeutung kommt dem erfindungsgemäßen Verfahren zu, wenn man zur Herstellung einer überwiegend D-Ribose enthaltenden Lösung eine Arabinose enthaltende Lösung epimerisiert, da einerseits die Ribose ein besonders begehrter Zucker ist, und andererseits bei dieser Epimerisierung der Einfluß auf das Epimerisierungsgleichgewicht besonders groß ist.

Gegenstand der Erfindung ist daher auch ein Verfahren zur Herstellung einer überwiegend D-Ribose enthaltenden Lösung bzw. von Reinribose durch Epimerisieren einer D-Arabinose enthaltenden Lösung in Gegenwart einer Molybdän(VI)-Verbindung bei erhöhter Temperatur, das dadurch gekennzeichnet ist, daß man die Epimerisierung in Gegenwart von 30 bis 200 Mol.%, bezogen auf die eingesetzte D-Arabinose, an einem gegebenenfalls Kristallwasser enthaltendem Metallsalz der Formel III

$MeX_2$ (III),

in der Me für Mg, Ca, Sr, Ba oder Zn, vorzugsweise Ca oder Sr, steht und X für Cl oder Br steht, und von 2 bis 20 Mol.%, bezogen auf den eingesetzten Zucker, durchführt und aus der hierbei erhaltenen Reaktionslösung den Molybdän-Katalysator und das Metallsalz durch Zugabe eines Alkali-oder Ammoniumcarbonats als schwerlöslichen Niederschlag ausfällt oder aber die bei der Epimerisierung erhaltene Reaktionslösung in Form einer wäßrigen Lösung direkt an einem stark sauren Kationenaustauscher, dessen Harz mit den bei der Epimerisierung verwendeten Metallionen beladen ist, chromatographisch fraktioniert.

Das erfindungsgemäße Epimerisierungsverfahren ist anwendbar für folgende Epimerisierungsgleichgewichte:

1) Arabinose (IIa) → Ribose (Ia)
2) Xylose (IIb) → Lyxose (Ib)
3) Glucose (IIb) → Mannose (Ib)
4) Galactose (IIb) → Talose (Ib)
5) Altrose (IIa) → Allose (Ia)
6) Idose (IIa) → Gulose (Ia) wobei die Epimerisierung von Altrose und Idose weniger wichtig ist, da diese Zucker selbst nur schlecht zugänglich sind.

Als Metallsalze der Formel III kommen die Chloride und Bromide von Erdalkalimetallen und Zink in Betracht. Die Chloride werden bevorzugt. Ganz besonders geeignet sind Calcium-und Strontiumsalze, insbesondere Calcium-und Strontiumchlorid. Wichtig ist, daß die Salze in dem Epimerisierungsgemisch homogen gelöst sind.

Als bestes Lösungsmittel für Salze ist Wasser bekannt. In Wasser als Lösungsmittel wird beispielsweise für die Epimerisierung von Arabinose eine Riboseselektivität von bis zu 45 % erreicht. Wird ein nichtwäßriges Lösungsmittel mitverwendet, wird eine noch weiter verbesserte Riboseselektivität von bis zu 75 % erreicht. Von den für solche Epimerisierungen bekannten Lösungsmitteln sind nur solche geeignet, in denen das System Zucker-Metallsalz-Molybdat homogen löslich ist. Geeignete Lösungsmittel sind insbesondere Methylalkohol, Ethylalkohol, Ethylenglykol sowie Ethylenglykolmonomethylether, vorzugsweise Ethanol, ggf. im Gemisch mit Wasser. Die Menge des dem Lösungsmittel zuzusetzenden Wassers ist u.a. abhängig von der Art des Metallsalzes. Bei Verwendung von Magnesiumchlorid als Metallsalz und Ethanol als Lösungsmittel kann man beispiels weise auf einen Wasserzusatz völlig verzichten. Bei Verwendung von Calcium-bzw. Strontiumchlorid dagegen muß das Ethanol etwa 5 bis 25 Gew.% Wasser enthalten, bei Verwendung von Bariumchlorid sogar bis zu 50 %. Die Menge des Lösungsmittels soll das 5-bis 20-fache der Menge an eingesetzter Arabinose nicht übersteigen. Dadurch arbeitet man bei dem erfindungsgemäßen Verfahren im allgemeinen mit Lösungen, die die Metallhalogenide in Konzentrationen von etwa 5 bis 40 %, vorzugsweise 10 bis 20 Gew.% enthalten. Es war überraschend, daß sich derartige Mengen an Salzen in den wasserhaltigen organischen Lösungsmitteln lösen.

Als Molybdän(VI)-Verbindung können für die erfindungsgemäße Epimerisierung die hierfür bekannten Verbindungen, wie Molybdäntrioxid, Molybdänsäure, Ammoniummolybdat, Kaliummolybdat, Natriummolybdat, Calciummolybdat oder Acetylacetonmolybdat verwendet werden. Bevorzugt werden Molybdänsäure, Ammoniummolybdat und Calciummolybdat. Von diesen Molybdänverbindungen werden 2 bis 20 Mol.%, vorzugsweise etwa 10 Mol.%, bezogen auf den eingesetzten Zucker, verwendet.

Es war überraschend, daß aus der Reaktionslösung kein Molybdat ausgefällt wird, obwohl das Metallhalogenid III in einem bis zu 20-fachen molaren Überschuß, bezogen auf $H_2MoO_4$ vorliegt.

Der optimale pH-Bereich für die Epimerisierung wurde bereits von Bilik mit pH = 2-4 angegeben. Bei der erfindungsgemäß bevorzugten Verwendung von etwa äquimolaren Mengen an $CaCl_2{\bullet}6H_2O$ und Arabinose in Ethanol/Wasser (95/5) liegt bereits ein optimaler pH-Bereich im Reaktionsgemisch vor. Ist eine pH-Korrektur notwendig, kann sie mit $NH_3$ leicht vorgenommen werden.

Die Reaktion kann durch Erhitzen zum Sieden unter Rückfluß durchgeführt werden, wenn ein Lösungsmittel mit einem Siedepunkt unterhalb von 90°C verwendet wird, oder bei Temperaturen von 78 bis 95°C, falls das Lösungsmittel einen Siedepunkt von 78°C oder oberhalb von 78°C hat. Die Reaktion kann unter atmosphärischem Druck durchgeführt werden oder bei leichtem Überdruck, wenn das Lösungsmittel einen Siedepunkt von kleiner als 78°C hat. Die Reaktionszeit beträgt 60 bis 180 Minuten.

Die entsprechend der Erfindung hergestellte D-Ribose-haltige Lösung, enthält zusätzlich zu D-Ribose und D-Arabinose, ganz geringe Mengen an D-Xylose und D-Lyxose, ein Molybdänsäure-Ion und $MeX_2$. Weiterhin kann sie Ionen und Nebenprodukte enthalten, die sich durch die Reaktion von Lösungsmittel und verwendeten Materialien ergeben.

Überraschenderweise lassen sich aus diesem Reaktionsgemisch die Metallsalze und die Molybdän-Verbindung gemeinsam durch Zugabe eines Alkali-oder Ammoniumcarbonats als schwerlöslicher Niederschlag ausfällen ohne daß erwähnenswerte Mengen an Zuckern mit ausfallen. Der Niederschlag entspricht einem Gemisch aus Carbonaten und Molybdaten der eingesetzten zweiwertigen Metalle. Er kann in HCl gelöst und zur Epimerisierung wiederverwendet werden.

Das Alkali-oder Ammoniumcarbonat verwendet man im allgemeinen in etwas weniger als äquimolaren Mengen, d.h. etwa 0,9 Mol, bezogen auf eingesetztes Metallsalz der Formel III. Die Summe der molaren Mengen an Carbonat und Molybdat sollen etwa den molaren Mengen des zu fällenden Metallsalzes III entsprechen.

Das Filtrat enthält neben den Zuckern noch Neutralsalze (vorwiegend Alkali-bzw. Ammoniumchloride). Letztere können recht vorteilhaft durch Elektrodialyse einer überwiegend wäßrigen Reaktionslösung abgetrennt werden. Man verwendet hierzu handelsübliche Elektrodialysegeräte.

Nach dem Entfernen der Neutralsalze kann die unumgesetzte Arabinose in an sich bekannter Weise, beispielsweise durch Einengen und Versetzen mit einem niederen Alkohol, weitgehend auskristallisiert werden, wodurch man ein mindestens 90 %iges Riboseöl erhalten wird, welches als solches für die Weiterverarbeitung zu Vitamin $B_2$ eingesetzt werden kann.

Es wurde weiter gefunden, daß man das bei der Epimerisierung erhaltene rohe Reaktionsgemisch in Form einer wäßrigen Lösung an einem stark sauren Kationenaustauscher, dessen Harz mit den bei der Epimerisierung verwendeten Metallionen beladen ist, sehr vorteilhaft chromatographisch fraktionieren kann. Es war überraschend, daß auf dem mit $Me^{2+}$-beladenen Kationenaustauscher auch die Molybdänverbindung quantitativ abgetrennt werden kann, ohne daß sich auf dem Austauscherharz ein schwerlösliches $MeMoO_4$ bildet. Nach dem Stand der Technik wurde die Molybdänverbindung aus dem Reaktionsgemisch mit Hilfe eines Anionenaustauschers abgetrennt, bevor die Trennung des Produktgemisches durch einen mit $Ca^{2+}$-beladenen Kationenaustauscher vorgenonnen wurde (vgl. JA-OS 57 054-197). Wird die Epimerisierung in wäßriger Lösung durchgeführt, dann kann die rohe Reaktionslösung direkt chromatographiert werden. Wird in einem organischen Lösungsmittel epimerisiert, muß dieses zuerst gegen Wasser ausgetauscht werden. Durch eine solche chromatographische Trennung kann beispielsweise das bei der Epimerisierung von Arabinose anfallende Gemisch in folgende Fraktionen getrennt werden:

1. Fraktion: enthaltend $MeX_2$; Molybdat und Nebenprodukte
2. Fraktion: enthaltend Arabinose, $MeX_2$ und Molybdat
3. Fraktion: enthaltend Ribose (> 99 %).

Aus Fraktion 1 kann man mit einem Alkali-oder Ammoniumhydroxid die Molybdänverbindung als schwer lösliches Molybdat ($MeMoO_4$) bzw. mit einem Alkali-oder Ammoniumcarbonat die Molybdänverbindung zusammen mit den Erdalkali-oder Zinkkationen als schwerlösliches Molybdat-Carbonat-Gemisch ausfällen. Die Molybdän(VI)-Verbindung wird dabei zu mindestens 97 % ausgefällt und kann leich durch Behandeln mit verdünnter HCl-Lösung wieder in die ursprüngliche, für die Katalyse aktive Form überführt werden.

Fraktion 2 kann nach dem Einengen direkt wieder in die Epimerisierung zurückgeführt werden.

Fraktion 3 enthält reine Ribose, die als Lösung weiterverarbeitet oder in bekannter Weise als Reinribose isoliert werden kann.

Bei stärkerer Belastung des Kationenaustauschers kann noch eine weitere Fraktion auftreten, die Arabinose im Gemisch mit Ribose enthält. Auch diese Fraktion kann - nach dem Einengen - wieder in die Epimerisierung eingeschleust werden oder aber - bei höheren Ribosegehalten - durch Einengen und Behandeln mit Alkoholen von Arabinose befreit werden. Die optimale Austauscherbelastung kann jeweils in Vorversuchen leicht ermittelt werden.

Für die chromatographische Trennung verwendet man im allgemeinen handelsübliche stark saure Kationenaustauscher des Typs Polystyrol/Divinylbenzol, das 4 bis 8 % Divinylbenzol enthält. Es sollte einen möglichst kleinen Teilchendurchmesser aufweisen. Man arbeitet vorzugsweise mit einem Austauscherharz, das eine Teilchengröße von 200 bis 400 Mesh, 100 bis 200 Mesh oder 50 bis 100 Mesh aufweist. Genannt seien handelsübliche stark saure Kationenaustauscher wie Dowex 50W-X4, Dowex 50W-X8; Lewatite TSW 40, Amberlite-CG-120 II und Mitsubishi MCI-Gel CK08P.

Mit Hilfe des erfindungsgemäßen Verfahrens gelingt es, bestimmte Pentosen und Hexosen mit sehr gutem Epimerisierungsgrad in die entsprechenden Zucker mit mehr cis-ständigen OH-Gruppen zu überführen. Sowohl die Zucker als auch der Molybdän(VI)-Katalysator und wahlweise auch die mitverwendeten Metallsalze können praktisch quantitativ zurückgewonnen werden.

Als besondere Vorteile der Erfindung sollte folgendes hervorgehoben werden:

a) Durch Zusatz von $MeX_2$ wird das Epimerisierungsgleichgewicht im gewünschten Sinne verschoben.

b) Die Metallsalze $MeX_2$ sind sehr billig, insbesondere das besonders wirksam anwendbare Calciumchlorid.

c) Der Metallsalzzusatz kann nach der Epimerisierung ohne Zuckerverlust abgetrennt werden.

d) Das Metallsalz $MeX_2$ hält den Ionenaustauscher bei der chromatographischen Trennung immer in einem für die Trennung optimalen Beladungszustand an $Me^{2+}$-ionen.

e) Der Überschuß an $MeX_2$ ermöglicht eine quantitative Rückgewinnung der Molybdänverbindung.

f) Sowohl bei der Abtrennung der Metallsalze $MeX_2$ als auch bei der des Katalysators treten keine nennenswerten Zuckerverluste auf.

Speziell für die technisch besonders bedeutungsvolle Epimerisierung von Arabinose zu Ribose ergeben sich durch das erfindungsgemäße Verfahren folgende Vorteile:

Man erhält Ribose in einer Selektivität von 75 % in einer Reaktionslösung, aus der sich

a) in einem Verfahrensschritt reine D-Ribose und nicht umgesetzte D-Arabinose separat und verlustfrei erhalten lassen, und der Molybdän-Katalysator und das Metallsalz in einer separaten Fraktion anfallen, aus der wahlweise entweder der Katalysator allein oder der Katalysator zusammen mit dem Metallsalz abgetrennt und wiederverwendet werden können

und

b) das Metallsalz und der Katalysator durch einfache Fällung quantitativ abgetrennt werden können und damit einer Wiederverwendung zugänglich sind und außerdem nach einer Arabinosefällung eine mindestens 90 %ige Riboselösung erhalten werden kann.

Sowohl die nach a) als auch die nach b) hergestellte Lösung eignet sich zur Herstellung von reinem N-D-Ribityl-3,4-xylidin (Ribamin), einer Vorstufe von Vitamin $B_2$ (Riboflavin). Mit nach a) aufgearbeiteter Ribose wird die maximale Ausbeute an reinem Ribamin erhalten (88 bis 90 %). an reinem Ribamin erhalten (88 bis 90 %).

Das Verhältnis der Pentosen wurde nach Fällung der Metallsalze III mit konz. $Na_2CO_3$-Lösung über HPLC bestimmt (Carbohydrate (Latek), Acetonitril/$H_2O$ = 88/12 + 0,5 % $H_3PO_4$, 1,5 ml/Min. 2 Säulen hintereinander). Es wird in folgender Reihenfolge eluiert:

Ribose 8,3 Min.
Lyxose 10,4 Min.
Xylose 11,4 Min.
Arabinose 12,7 Min.

Die Bestimmung der reduzierenden Zuckern erfolgte über Reduktion von $CuSO_4$ in alkalischer, citronensäurehaltiger Lösung zu CuO und anschließender iodometrischer Bestimmung.

Die Erdalkali-bzw. Zinkionen wurden komplexometrisch bestimmt, Mo wurde über Atomabsorptionsspektroskopie ermittelt.

Beispiel 1

Eine Lösung von 15 g Arabinose, 40,6 g $MgCl_2 \bullet 6\ H_2O$ und 1,8 g Ammoniummolybdat in 80 ml Ethanol wurde 3 Stunden (h) unter Rückfluß zum Sieden erhitzt. Die HPLC-Analyse zeigt

Ribose 62 %
Lyxose nicht erkennbar
Xylose nicht erkennbar
Arabinose 38 %

Beispiel 2

Eine Lösung von 15 g Arabinose, 27 g $SrCl_2 \bullet 6\ H_2O$ und 1,8 g Ammoniummolybdat in einem Gemisch aus 80 ml Ethanol und 10 ml Wasser wurde 3 h unter Rückfluß zum Sieden erhitzt. Nach dem Abkühlen auf 40°C wurde mit gesättigter $Na_2CO_3$-Lösung versetzt, über Nacht gerührt, der Niederschlag abfiltriert und gründlich mit $H_2O$ gewaschen. In dem erhaltenen Filtrat wurde durch HPLC-Analyse ermittelt:

Ribose 70,9 %
Arabinose 29,1 %
Xylose nicht erkennbar
Lyxose nicht erkennbar.

Beispiel 3

Eine Lösung von 15 g Arabinose, 44 g $CaCl_2 \bullet 6\ H_2O$ und 1,7 g Molybdänsäure in 80 ml Ethanol und 20 ml Wasser wurde mit $NH_3$ auf einen pH-Wert von 3,0 eingestellt und anschließend 3 h unter Rückfluß zum Sieden erhitzt. Nach dem Abkühlen wurden 21,2 g $Na_2CO_3$ in 45 g $H_2O$ zugesetzt. Man rührte über Nacht, saugte über eine Nutsche ab und wusch den Niederschlag gründlich mit Wasser. Man erhielt 21,5 g $CaCO_3 \bullet CaMoO_4$ und eine Ausbeute an reduzierendem Zucker von 95 % der Theorie.

Die HPLC-Analyse der Zuckerlösung ergab:

Ribose 68 %
Lyxose 0,6 %
Arabinose 31,4 %
Xylose nicht erkennbar.

Beispiel 4

Eine Lösung von 15 g Arabinose, 21,9 g $CaCl_2 \bullet 6\ H_2O$, 1,7 g Molybdänsäure in 80 ml Glykolmonomethylester und 20 ml $H_2O$ wurde mit $NH_3$ auf einen pH-Wert von etwa 3,0 gebracht und 60 Minuten bei 90°C gerührt. Nach der Fällung mit $(NH_4)_2CO_3$ wurde bei einer Gesamtausbeute von 96 % an reduzierenden Zuckern erhalten:

Ribose 65 %
Lyxose 0,8 %
Arabinose 34,2 %
Xylose nicht erkennbar.

Beispiel 5

Eine Lösung von 15 g Arabinose, 35,32 g $SrCl_2 \bullet 6\ H_2O$ und 1,8 g Ammoniummolybdat in einer Mischung aus 20 ml Wasser und 80 ml Ethanol wurde 3 h unter Rückfluß zum Sieden erhitzt. Nach dem Abkühlen wurde das Verhältnis der Pentosen durch HPLC ermittelt.

Ribose: 69,6 %
Lyxose: 0,3 %
Arabinose: 30,1 %

Beispiel 6

Eine Lösung von 30 g Arabinose, 44 g $CaCl_2 \bullet 6\ H_2O$ und 3,6 g Ammoniummolybdat wurde in 190 ml Ethanol und 8 ml $H_2O$ 3 h unter Rühren und Rückflußkühlung zum Sieden erhitzt. Anschließend kühlte man auf 50°C ab und tauschte das Ethanol gegen 50 % der Menge an $H_2O$ aus. Die Ausbeute an reduzierendem Zucker betrug 95 %.

Ribose 75,3 %
Lyxose 0,4 %
Arabinose 24,3 %

Nach Chromatographie an dem stark sauren Ionenaustauscher Dowex 50W-X4 in der $Ca^{2+}$-Form wurden 4 Fraktionen erhalten, wobei die Ribose/Arabinose-Mischfraktion (Fraktion 3) nach dem Einengen erneut chromatographiert wurde.

Fraktion 1 $CaCl_2$ 63 %
Mo 55 %
Nebenprodukte

Fraktion 1 wurde mit NaOH, $NH_3$ bzw. KOH auf einen pH-Wert von etwa 8,5 bis 9 gebracht und 1 h auf 100°C erhitzt. Der Niederschlag wurde abfiltriert. Man erhielt 2,2 g $CaMoO_4$ (~ 98 %).

Fraktion 2

| | | |
|---|---|---|
| Lyxose | 1,6 % | |
| Xylose | 0,4 % | |
| Arabinose | 97,9 % | |
| Ribose | 1,0 % | |
| | 7,1 g | reduzierende Zucker |
| | ≙ 23,7 % | bezogen auf eingesetzte Arabinose |
| Ca | 38 % | |
| Mo | 45 % | |

Fraktion 2 wurde eingeengt und in Beispiel 7 eingesetzt.

Fraktion 4

| | | |
|---|---|---|
| Ribose | 99,7 % | |
| Arabinose | 0,3 % | |
| | 21,4 g | reduzierende Zucker |
| | ≙ 71,2 % | bezogen auf eingesetzte Arabinose |

Beispiel 7

a) Eine Lösung aus 23 g Arabinose und 7 g Zucker aus Fraktion 2 von Beispiel 6, 28 g $CaCl_2 \bullet 6\ H_2O$ und etwa 12 g $CaCl_2 \bullet 6\ H_2O$ aus Fraktion 2 von Beispiel 6, ferner 2,5 g $CaMoO_4$ in 15 ml 1 n HCl gelöst und etwa 1,6 g Ammoniummolybdat aus Fraktion 3 von Beispiel 6 in 190 ml Ethanol wurde 3 h unter

Rückfluß zum Sieden erhitzt. Die Ausbeute an reduzierenden Zuckern betrug 93 % folgender Zusammensetzung:

Ribose 68,5 %
Lyxose 0,9 %
Xylose 0,2 %
Arabinose 30,4 %

Die Aufarbeitung erfolgte analog Beispiel 6. Es wurden 19 g Ribose, (99,8 %ig) erhalten, was einer Gesamtausbeute an 63,4 % entspricht.

b) Als Fraktion 1 wurden ca. 520 g erhalten, die 11 mmol Molybdat enthielten. Diese Fraktion wurde mit 12 mmol einer 50 %igen wäßrigen NaOH versetzt und 1 h unter Rückfluß zum Sieden erhitzt. Nach dem Abkühlen wurde der erhaltene Niederschlag abfiltriert und getrocknet. Man erhielt 2,38 g $CaMoO_4$, (Molybdängehalt = 44,2 %). Das entspricht einer Abtrennung von Mo zu 99,7 % der Theorie. Der Restgehalt an Molybdän im Filtrat war kleiner als 1 ppm.

Beispiel 8

2,25 g des gemäß Beispiel 7b gewonnenen $CaMoO_4$ wurden mit 15 ml 1m wäßriger HCl versetzt und 30 Minuten unter Rückfluß zum Sieden erhitzt. Anschließend wurden hierzu 15 g Arabinose, 14,1 g $CaCl_2 \bullet 2H_2O$ und 90 ml Ethanol addiert und das erhaltene Reaktionsgemisch 3 h zum Sieden erhitzt.

Danach enthielt es 74,9 % Ribose, 25,1 % Arabinose, keine Lyxose und keine Xylose.

Beispiel 9

Eine Lösung aus 30 g Xylose, 43,8 g $CaCl_2 \bullet 6H_2O$ und 3,6 g Ammoniummolybdat in 190 ml Ethanol wurde unter Rühren 1 h unter Rückflußkühlen zum Sieden erhitzt. Anschließend kühlte man auf 55°C ab und destillierte das Ethanol unter vermindertem Druck ab, wobei gleichzeitig 100 ml Wasser zudosiert wurden. Die Ausbeute an reduzierenden Zuckern betrug 96 %; die Lösung enthielt 63 %. Lyxose und 37 % Xylose. Bei der anschließenden Chromatographie an Dowex 50W-X4 wurden 3 Fraktionen erhalten, die die im folgenden erläuterten Bestandteile enthielten:

Fraktion 1: Mo (VI) und $CaCl_2$
Fraktion 2: Mo (VI), $CaCl_2$; 87 % Xylose, 13 % Lyxose (insgesamt 10 g reduzierende Zucker)
Fraktion 3: 93 % Lyxose, 7 % Xylose (insgesamt 17,8 g reduzierende Zucker).

Beispiel 10

36 g Galactose, 43,8 g $CaCl_2 \bullet 6\ H_2O$ und 3,6 g Ammoniummolybdat wurden in einer Mischung aus 4 g Wasser und 190 ml Ethanol gelöst und die Lösung 3 h unter Rückfluß zum Sieden erhitzt. Anschließend wurde das Ethanol bei 55°C unter vermindertem Druck abdestilliert, wobei gleichzeitig 100 ml Wasser zudosiert wurden. Das Reaktionsgemisch enthielt 52,2 % unumgesetzte Galactose und 47,8 % Talose.

Durch chromatographische Trennung an Dowex 50 W-X4 in der $Ca^{2+}$-Form analog Beispiel 7 wurden 15,1 g einer 99,9 %igen Talose erhalten.

Beispiel 11

Analog Beispiel 10 wurde eine Lösung von 36 g Glucose, 43,8 g $CaCl_2 \bullet 6\ H_2O$ und 3 g Ammoniummolybdat in 4 ml Wasser und 190 ml Ethanol umgesetzt. Nach 3-stündigem Sieden wurde ein Glucose/Mannose-Verhältnis von 45/55 ermittelt.

Beispiel 12

Eine Lösung von 150 g Arabinose, 219 g $CaCl_2 \bullet 6 H_2O$ und 36 g Ammoniummolybdat in 900 ml Ethanol und 50 ml $H_2O$ wurde 3 h unter Rückfluß zum Sieden erhitzt. Nach Abkühlen auf 50°C wurde mit 300 ml einer 33 %igen $Na_2CO_3$-lösung versetzt, über Nacht gerührt und der Niederschlag abfiltriert. Aus dem Filtrat wurde unter vermindertem Druck die Hauptmenge an Ethanol abdestilliert und Wasser zugesetzt. Aus der erhaltenen wäßrigen Lösung wurden die Neutralsalze (im wesentlichen NaCl) durch Elektrodialyse in einer Standard-Elektrodialyse-Laborzelle mit 10 Entsalzungseinheiten entsalzt. Es wurde eine salzfreie dunkelgelbe Lösung erhalten, die 138 g reduzierende Zucker (92 %) enthielt. Diese Lösung wurde weitgehend eingedampft, mit Ethanol versetzt und 16 h gerührt. Das Kristallisat (27 g Arabinose/Ribose = 90:10) wurde abgesaugt, mit Ethanol gewaschen. Das Filtrat enthielt:

Ribose 91 %
Lyxose 1 %
Arabinose 8 %
Mo 0,05 mmol
$Ca^{2+}$ nicht nachweisbar.

**Ansprüche**

1. Verbessertes Verfahren zur Epimerisierung von Pentosen und Hexosen durch Erhitzen von in einem Lösungsmittel gelöstem Zucker in Gegenwart einer Molybdän(VI)-Verbindung, dadurch gekennzeichnet, daß man zur Herstellung eines Zuckers der Formeln Ia oder Ib mit cis-ständigen OH-Gruppen in 2-und 3-Stellung des Zuckers

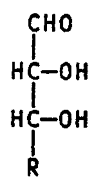
(Ia)

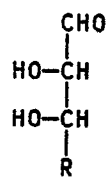
(Ib)

in der R für einen der Reste

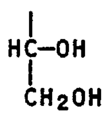
,
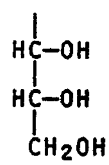
oder
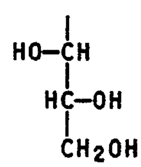

steht,
eine homogene Lösung des entsprechenden Zuckers der Formeln IIa oder IIb mit trans-ständigen OH-Gruppen in 2-und 3-Stellung des Zuckers

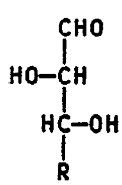
(IIa)

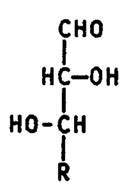
(IIb)

in Gegenwart von 30 bis 200 Mol.%, bezogen auf den eingesetzten Zucker, an einem gegebenenfalls Kristallwasser enthaltenden Metallsalz der Formel (III)

$MeX_2$ (III),

in der Me für Mg, Ca, Sr, Ba und Zn steht und X für Cl oder Br steht, und in Gegenwart von 2 bis 20 Mol.%, bezogen auf den eingesetzten Zucker einer Molybdän(VI)-Verbindung auf Temperaturen von 75 bis 100°C erhitzt.

2. Verfahren gemäß Anspruch 1, dadurch gekennzeichnet, daß man aus der erhaltenen Reaktionslösung den Molybdän-Katalysator und das Metallsalz durch Zugabe eines Alkali-oder Ammoniumcarbonats als schwer löslichen Niederschlag ausfällt.

3. Verfahren gemäß Anspruch 2, dadurch gekennzeichnet, daß man aus der Reaktionslösung nach dem Abtrennen des schwerlöslichen Niederschlages und nach Entfernen der Alkali-bzw. Ammoniumchloride in bekannter Weise durch Elektrodialyse den unumgesetzten Zucker in an sich bekannter Weise auskristallisiert.

4. Verfahren gemäß Anspruch 1, dadurch gekennzeichnet, daß man die Epimerisierung in Gegenwart von etwa 80 bis 120 Mol.%, bezogen auf eingesetzten Zucker, an dem Metallsalz der Formel III durchführt.

5. Verfahren gemäß Anspruch 1, dadurch gekennzeichnet, daß man die Epimerisierung in Gegenwart von 8 bis 12 Mol.%, bezogen auf den eingesetzten Zucker, einer Molybdän(VI)-verbindung durchführt.

6. Verfahren gemäß Anspruch 1, dadurch gekennzeichnet, daß man die Epimerisierung in Gegenwart eines Calcium-oder Strontiumsalzes der Formel III durchführt.

7. Verfahren gemäß Anspruch 1, dadurch gekennzeichnet, daß die Epimerisierung in ggf. bis zu 50 % Wasser enthaltendem Ethanol als Lösungsmittel durchgeführt wird.

8. Verfahren gemäß Anspruch 1, dadurch gekennzeichnet, daß man das bei der Epimerisierung erhaltene rohe Reaktionsgemisch in Form einer wäßrigen Lösung an einem stark sauren Kationenaustauscher, dessen Harz mit den bei der Epimerisierung verwendeten Metallionen beladen ist, chromatographisch fraktioniert.

9. Verfahren gemäß Ansprüche 1 bis 8, dadurch gekennzeichnet, daß man zur Herstellung von einer überwiegend D-Ribose enthaltenden Lösung eine D-Arabinose enthaltende Lösung epimerisiert.

10. Verfahren zur Herstellung einer überwiegend D-Ribose enthaltenden Lösung bzw. von Reinribose durch Epimerisierung einer D-Arabinose enthaltenden Lösung in Gegenwart einer Molybdän(VI)-Verbindung bei erhöhter Temperatur, dadurch gekennzeichnet , daß man die Epimerisierung in Gegenwart von 30 bis 200 Mol.%, bezogen auf die eingesetzte D-Arabinose an einem ggf. Kristallwasser enthaltendem Metallsalz der Formel III

$MeX_2$ (III),

in der Me für Mg, Ca, Sr, Ba oder Zn steht und X Cl oder Br bedeutet, und von 2 bis 20 Mol.%, bezogen auf die eingesetzte Arabinose, einer Molybdän(VI)-Verbindung durchführt und aus der hierbei erhaltenen Reaktionslösung den Molybdän-Katalysator und das Metallsalz durch Zugabe eines Alkali-oder Ammoniumcarbonats als schwerlöslichen Niederschlag ausfällt oder aber die bei der Epimerisierung erhaltene Reaktionslösung direkt an einem stark sauren Kationenaustauscher, dessen Harz mit den bei der Epimerisierung verwendeten Metallionen beladen ist, chromatographisch fraktioniert.